# EUROPEAN PATENT APPLICATION

(11) **EP 1 980 258 A1**
(43) Date of publication of application: **15.10.2008**
(21) Application number: 06712241.6
(22) Date of filing: 24.01.2006
(51) Int. Cl.: A61K 31/7004, A23F 5/24, A23L 1/30, A23L 2/52, A61K 31/7016, A61K 31/702, A61P 9/00, C07H 3/00, C08B 37/14

(54) **COMPOSITION HAVING EFFECT OF LOWERING BLOOD PRESSURE AND/OR INHIBITING INCREASE IN BLOOD PRESSURE AND FOOD AND DRINK CONTAINING THE SAME**

(71) Applicant: Ajinomoto General Foods, Inc., Shinjuku-ku Tokyo 163-1440 (JP)
(72) Inventor: TAKAO, Izumi, Mie 513-0046 (JP); ASANO, Ichiro, Tokyo 184-0011 (JP); FUJII, Shigeyoshi, Mie 513-0807 (JP); KANEKO, Masayuki, Tokyo 182-0001 (JP); NIELSON, John R., Victoria 3207 (AU); STEFFEN, Daniel G., New Jersey 07936 (US); HATZOLD, Thomas, Munchen 81737 (DE)
(74) Representative: Smaggasgale, Gillian Helen
(86) International application number: PCT/JP2006/301025
(87) International publication number: WO 2007/086100

(57) **Abstract**

The present invention regards the composition having a blood pressure reducing and/or elevation suppressing effect comprising a mannose-based oligosaccharide mass comprising 1 to 10 molecules of monosaccharides linked together, said molecules mainly comprising mannose. It is the object of the present invention to provide an economical and simple food and drink which has an excellent blood pressure reducing and/or elevation suppressing effect without changing ordinary eating habits much, and a composition ingestible with a food and drink which effectively uses waste resources such as coffee extraction residues.

## Description

### [Technical Field]

The present invention relates to a composition having a blood pressure reducing and/or elevation suppressing effect which comprises, as the main component, oligosaccharides composed of constituent monosaccharides mainly comprising mannose and an oral ingestible composition using the composition, particularly a food and drink. Further, the present invention relates to effective utilization of unused resources.

### [Background Art]

Heretofore, coffee extraction residues have been mostly incinerated or disposed as industrial waste. In recent years, the coffee residues have been used as raw materials for compost or active carbon but such use cannot be said to be sufficient from the standpoint of high utilization of unused resources, and establishment of a method of high utilization of coffee extraction residues is an important requirement.

Lifestyle-related diseases on the rise recently such as high blood pressure, diabetes and hyperlipemia are said to be closely related to dietary habits, and therefore, prevention of such diseases by improving dietary habits is emphasized. High blood pressure is a condition which occurs in about 50% of elderly people (60 years and above) in Japan. It is well known that if high blood pressure is left untreated, stroke, cardiac infarction or cardiac hypertrophy caused by sclerosis of the arteries is induced. A therapeutic drug for treating high blood pressure includes a diuretic agent, a sympathetic blocking agent, a vasodilatory agent, and so on, The positive effect of using such drugs is expected to be high, but on the other hand, adverse effects such as edema, polyuria and palpitation could be experienced by patients, which is a major drawback when using the drugs to treat high blood pressure.

For this reason, non-drug preventative methods such as improvement in lifestyle, diet following an exercise regime, and limiting the intake of alcohol and tobacco are recommended.
In particular, improvement in everyday dietary life is emphasized because an improvement of diet alone can not only prevent high blood pressure but also suppress the progression of the condition. Therefore, efforts have been made to identify substances in food that are beneficial in preventing high blood pressure, and the mechanism of action of these substances is studied for example, in patent document 1 and 2. However, food or drink with additives of special substances impairs the original taste of the food or drink. Moreover, food or drink takes long until exerting effect. Even though the applicant of the present application has already found that a mannose-based oligosaccharide mass comprising 1 to 10 molecules of monosaccharides linked together, said molecules mainly comprising mannose and a small quantity of other substances obtained from food comprising mannan such as hydrolysates of coffee extraction residues have controlling intestinal functions accompanied by growth of bifidobacteria or educing serum lipid function, a blood pressure reducing and/or elevation suppressing effect has not studied yet.
The prior art of the present invention includes JP 2002-80354 A, and JP 2002-272420 A.

### [Disclosure of the Invention]

### [Problem to Be Solved by the Invention]

The present invention has an object to provide a simple and economical, and safe vegetable derived food and drink which is effective in reducing and lowering blood pressure g and/or having an elevation suppressing effect without much changing ordinary eating habits.

### [Means for solving The Problems]

As a result of serious investigations conducted by the present inventors in order to solve the aforementioned problems, it is found that food materials, containing a large amount of mannan, mainly from hydrolysates of coffee extraction residues, a mannooligosaccharide mass having a degree of polymerization of 1 to 10 and having a reduced content of monosaccharide residues other than a mannose residue in the chain of oligosaccharide molecules, that is, a mannose-based oligosaccharide mass comprising 1 to 10 molecules of monosaccharides linked together, said molecules comprising mannose, such as glucose and galactose, has a blood pressure reducing and/or elevation suppressing effect. Furthermore, it is found that by obtaining noncolored acid-free mannooligosaccharide mass having a degree of polymerization of 1 to 10 and having a reduced amount of monosaccharide residues other than a mannose residue in the chain of oligosaccharide molecules, the range of its application to food and drinks can be remarkably broadened.

Accordingly, embodiments of the present invention are as follows .
1. A composition having a blood pressure reducing and/or elevation suppressing effect which comprises a mannose-based oligosaccharide mass comprising 1 to 10 molecules of monosaccharides linked together, said molecules mainly comprising mannose.
2. The composition having a blood pressure reducing and/or elevation suppressing effect of item 1, wherein said mannose-based oligosaccharide mass comprising 1 to 10 molecules of mannose linked together.
3. The composition having a blood pressure reducing and/or elevation suppressing effect of item 1, wherein said molecules comprising mannose and at least one monosaccharide selected from the group consisting of glucose, galactose and fructose.
4. The composition having a blood pressure reducing and/or elevation suppressing effect of item 1, wherein the content ratio of said mannose-based oligosaccharide mass is not less than 60 weight% based on the total solid content in the composition.
5. The composition having a blood pressure reducing and/or elevation suppressing effect of any one of items 1 to 4, wherein the content ratio of a mannose residue in said mannose-based oligosaccharide mass is not less than 70 weight%.
6. The composition having a blood pressure reducing and/or elevation suppressing effect of any one of items 1 to 5, wherein said mannose-based oligosaccharide mass comprising 2 to 6 molecules of mannose units in the oligosaccharide molecules.
7. The composition having a blood pressure reducing and/or elevation suppressing effect of any one of items 1 to 6, wherein said mannose-based oligosaccharide mass is obtained by the hydrolysis treatment of mannan.
8. The composition having a blood pressure reducing and/or elevation suppressing effect of item 7, wherein said mannan is obtained from coffee beans and/or coffee extraction residues.
9. The composition having a blood pressure reducing and/or elevation suppressing effect of any one of items 1 to 8, wherein said mannose-based oligosaccharide mass is β-1,4-mannooligosaccharide mass.
10. An oral ingestible composition comprising a composition having a body fat reducing function of any one of items 1 to 9.
11. The composition of item 10, wherein said oral ingestible composition is a drink.
12. The composition of item 11, wherein said drink is a liquid coffee, an instant coffee or a coffee mixed drink.
13. The composition of item 10, wherein said oral ingestible composition is a food.

### [Effect of the Invention]

As a result of investigations of a blood pressure reducing and/or elevation suppressing effect, by the present inventors in order to solve the above described problem, with the use of the composition, obtained by the above described method, having a blood pressure reducing and/or elevation suppressing effect, and, comprising a mannose-based oligosaccharide mass comprising 1 to 10 molecules of monosaccharides linked together, said molecules mainly comprising mannose, it is found that a blood pressure reducing and/or elevation suppressing effect, is remarkably exhibited. Addition of a mannose-based oligosaccharide mass having a blood pressure reducing and/or elevation suppressing effect to food and drinks enables simple and economical ingestion in daily eating habits to expect the internal fat reduction effect. The mannose-based oligosaccharide mass having a blood pressure reducing and/or elevation suppressing effect can be available from waste such as coffee extraction residues, and accordingly such resources that have not been used heretofore can also be effectively utilized.

### [Brief Description of Drawing]

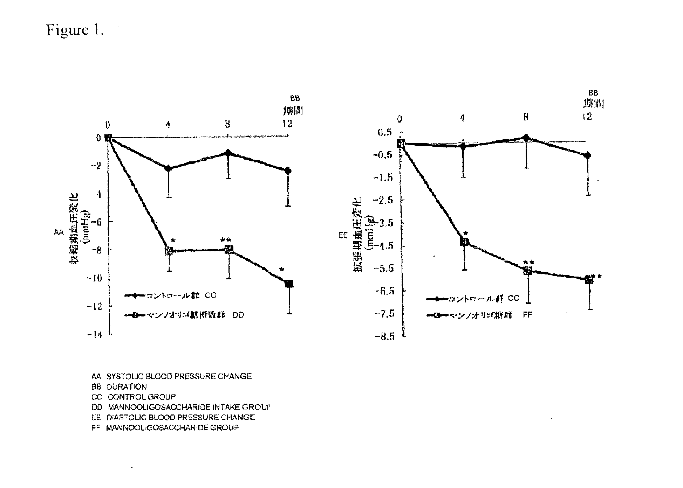

Fig. 1 shows graphs of blood pressure change of humans by mannooligosaccharide mass ingestion. (Data are expressed by (average) ± (standard deviation) (n=24) ^{*};p<0.05, ^{**};p<0.01, ^{***};p<0.001/There were significant difference compared to control group.)

### [Best Mode for Working the Invention]

The contents of the present invention will now be explained in detail.

In the present invention, the term "a mannose-based oligosaccharide mass" means a oligosaccharide mass comprising mannose (monosaccharide) as the main constituent element. The term "an oligosaccharide mass" as used herein generally means a substance which holds a position between a monosaccharide mass and a polysaccharide mass and is composed of a definite small amount of glycosyl bonds of monosaccharide molecules, in other words, a polymer having a comparatively small number of constituent monosaccharides. The term "an oligosaccharide mass" means a composition comprising a plurality of oligosaccharide molecules consisting of various numbers of constituent monosaccharides. And, the term "a mannose-based oligosaccharide mass" refers to a composition comprising a plurality of oligosaccharide molecules consisting of various types and numbers of constituent monosaccharides. In the present specification, the term "a mannooligosaccharide mass" is used in the same meaning as the term "a mannose-based oligosaccharide mass".

In the present invention, the degree of polymerization of an oligosaccharide is sometimes described as "DP". DP means the number of a monosaccharide constituting an oligosaccharide. In other words, mannose of a monosaccharide is represented by "DP 1", and a mannooligosaccharide constituted of four mannose units has a degree of polymerization of four, namely, is represented by "DP 4". From a technical viewpoint, the sugar having a degree of polymerization 1 (DP 1) is "a monosaccharide" and not an oligosaccharide. However, since an oligosaccharide mass (a mixture) used in the present invention sometimes contains monosaccharide molecules, even such oligosaccharide mass is generally called "a oligosaccharide mass" in the present specification. Namely, it should be understood that in the case of "a mannose-based oligosaccharide mass comprising 1 to 10 molecules of monosaccharides linked together", this mixture of oligosaccharides sometimes contains monosaccharide molecules having a degree of polymerization of 1.

The mannose-based oligosaccharide mass used in the present invention is preferably a composition of a plurality of types of oligosaccharides comprising 1 to 10 molecules of monosaccharides linked together, said molecules comprising mainly mannose. Particularly preferred is a composition of oligosaccharides comprising 1 to 10 molecules of mannose linked together or a composition of a plurality of types of oligosaccharides comprising 1 to 10 molecules of monosaccharides linked together, said molecules comprising mannose and at least one monosaccharide selected from the group consisting of glucose, galactose and fructose.

In the composition of the present invention, the total content ratio of the mannose-based oligosaccharide mass comprising 1 to 10 molecules of monosaccharides linked together is preferably not less than 60 weight%, more preferably not less than 80 weight% based on the total solid content.

In the saccharide composition of the composition of the present invention, it is desired that the ratio of a mannose residue is not less than 70 weight%, preferably not less than 80 weight%. Ratios of less than 70% of mannose residues cannot much effect blood pressure reducing and/or elevation suppressing, and, simultaneously, increase sweetness to tend to narrow the application range.

Further, it is preferred that the mannose-based oligosaccharide mass used in the present invention comprises the number of a mannose unit of 2 to 9, particularly 2 to 6 in the oligosaccharide molecules.

One embodiment of the present invention is a composition having a blood pressure reducing and/or elevation suppressing effect, and which comprises the above described mannose-based oligosaccharide mass. The term "composition having a blood pressure reducing and/or elevation suppressing effect" as used herein broadly means a composition having a function to generally reduce blood pressure or suppress elevation of blood pressure. Thus, the composition having a blood pressure reducing and/or elevation suppressing effect according to the present invention can be produced with the use of the oligosaccharide having the above described blood pressure reducing and/or elevation suppressing effect.

Another embodiment of the present invention is a food and drink having an indication that this food and drink contains a composition having a blood pressure reducing and/or elevation suppressing effect characterized by having a human blood pressure reducing and/or elevation suppressing effect, and , therefore being suited for eating and drinking by a person who is anxious about blood pressure.

The mannose-based oligosaccharide mass used in the present invention can be produced by hydrolyzing mannan. The raw material mannan herein can be obtained by extraction from, for example, a copra meal or copra flake obtained from a coconut, Huacra Palm of a South African Arecaceae (Palmae) plant, a yam mannan, a Chinese yam mannan. A sugar mixture can be obtained by treating the mannan thus obtained by at least one method selected from acid hydrolysis, high temperature thermal hydrolysis, enzyme hydrolysis and microorganism fermentation, and preferably purifying the resulting hydrolysate by the method such as active carbon treatment, adsorptive resin treatment, ion-exchange resin treatment and ion-exchange membrane treatment. This mixture contains the above described mannose-based oligosaccharide mass having a body fat reducing function. The composition thus obtained has a body fat reducing function of the present invention. Furthermore, the composition having a body fat reducing function of the present invention may be the one obtained by treating glucomannan present in a corn of konnyak, lily, narcissus, a lycoris and the like and galactomannan present in locust bean gum, guar gum and the like by at least one method selected from acid hydrolysis, high temperature thermal hydrolysis, enzyme hydrolysis and microorganism fermentation and then subjecting the resulting hydrolysate to purification treatment by the method such as active carbon treatment, adsorptive resin treatment, ion-exchange resin treatment and ion-exchange membrane treatment to increase the ratio of mannose as a constituent sugar.

Accordingly, mere "mannan" as used in the present specification broadly includes mannan of a polysaccharide having D-mannose alone as the constituent unit and, additionally, galctomannan and glucomannan of polysaccharides having mannose and galactose or glucoses as constituent units. Further, D-mannose is an aldohexose and an isomer of D-glucose differing only by having the opposite steric configuration of the hydroxyl group bonded to the carbon adjacent to the carboxyl group.

Furthermore, the composition having a blood pressure reducing and/or elevation suppressing effect of the present invention can be obtained by treating coffee raw beans or roasted coffee beans by at least one method selected from acid hydrolysis, high temperature thermal hydrolysis, enzyme hydrolysis and microorganism fermentation and purifying the resulting hydrolyzate by the method such as active carbon treatment, adsorptive resin treatment, ion-exchange resin treatment and ion-exchange membrane treatment. Or, the composition having a blood pressure reducing and/or elevation suppressing effect of the present invention may be obtained by treating spent coffee residues by at least one method selected from acid hydrolysis, high temperature thermal hydrolysis, enzyme hydrolysis and microorganism fermentation and purifying the resulting hydrolysate by the method such as active carbon treatment, adsorptive resin treatment, ion-exchange resin treatment and ion-exchange membrane treatment. In general, when roasted and ground coffee is extracted by a commercial extractor, at this instance, galactose of a side chain of a galactomannan present in the roasted coffee is solubilized and an arabinogalactan is solubilized by hydrolysis. Thus, it is presumed that mannan is rich in coffee residues and, in addition, mannan takes a straight-chain structure. On the other hand, cellulose is hard to decompose and remains in the residues but by suitably selecting conditions for specifically hydrolyzing mannan without decomposing cellulose, mannose-based oligosaccharide mass can be obtained.

Particularly, the methods of decomposing coffee extraction residues include a hydrolysis method with an acid and/or by high temperatures, a decomposition method with an enzyme and a decomposition method by microorganism fermentation and are not limited to them. The hydrolysis method with an acid and/or by high temperatures is disclosed in Japanese Patent Publication (A) Nos. Sho 61-96947 and Hei 02-200147 and the like. Spent coffee residues formed in the commercial multistage coffee extraction system can be hydrolyzed in a reaction vessel with addition of an acid catalyst or can be hydrolyzed without addition of the acid catalyst after short time high temperature treatment. It is convenient to use a tubular plug flow reactor and use any reactor suited for conducting a short time comparatively high temperature reaction can give a good result. By controlling reaction time and reaction temperature, mannan having DP 10 to DP 40 is solubilized and hydrolyzed to decompose to oligosaccharide mass of DP 1 to DP 10, and then the hydrolyzates are separated from residues to obtain mannooligosaccharide mass. Further, coffee extraction residues as used herein mean so-called coffee extraction grounds after atmospheric or pressurized extraction of roasted and ground coffee with a solvent such as water.

When the composition having a blood pressure reducing and/or elevation suppressing effect is obtained by the hydrolysis treatment of coffee beans (including roasted beans and roasted and ground coffee beans), the type of coffee beans and their place of production are not limited, and any coffee beans of Coffea Arabica, Coffea Robusta, Coffea Liveria or the like may be used, and coffee beans produced in countries such as Brazil and Columbia can be used, and one type of beans alone or a blend of two or more types of beans may be used. Even coffee beans of inferior quality which have been disposed due to noncommercial value or small beans can be used. Roasted and ground coffee beans (including various forms of coarse-grinding, middle-grinding, middle fine-grinding, fine-grinding or the like using an ordinary grinder or a mill) which are obtained by roasting the above described beans very slightly, slightly, intermediately or deeply with the use of a generally employed roaster (of a hot air system, a far infrared system, a charcoal fire system or the like).

Further, coffee extraction residues which can be used may be any extraction residue obtained after the extraction treatment of roasted and ground coffee using a conventional process for producing liquid coffee or instant coffee through atmospheric or pressurized extraction and independently of the origin of the coffee beans and the preparation method.

Herein, the above described hydrolysis treatment will explained in detail. As the enzyme decomposition method, coffee extraction residues are suspended in an aqueous medium and, for example, a commercially available cellulase or hemicellulase may be added thereto and suspended while stirring. The amount of enzyme, the temperature and other conditions may be those which are employed in conventional enzymatic reactions without problems, and the optimum amount of action of the enzyme used, the temperature, and other conditions and factors may be suitably selected.

As the microorganism fermentation method, for example, microorganisms which produce cellulase, hemicellulase or the like may be planted in coffee extract residues suspended in an aqueous medium and cultured. The microorganisms used may be bacteria, Basidiomycota or the like which produce an enzyme capable of decomposing mannan in the coffee extraction residues, and cultivation conditions and the like may be suitably selected depending on the microorganisms used.

The reaction solution containing a composition having a blood pressure reducing and/or elevation suppressing effect comprising a mannose-based oligosaccharide mass can be purified, if necessary. As the purification method, the reaction solution is decolorized/deodorized by boneblack, active carbon, the carbonization method, adsorptive resins, the magnesia method, the solvent extraction method or the like, then desalted and deacidified by ion-exchange resins, ion-exchange membranes, electrodialysis or the like. A combination of purification methods and purification conditions may be suitably selected depending on the amounts of a pigment, a salt, an acid and the like present in the reaction solution comprising a mannose-based oligosaccharide mass and other factors.

Further, the present invention relates to an orally ingestible composition comprising the above explained composition having an internal fat reduction action according to the present invention, particularly a food and drink. Furthermore, the composition having a blood pressure reducing and/or elevation suppressing effect, and according to the present invention can be used not only in the above described food and drink but also in the field of cosmetics, medicines and feed. The composition having a blood pressure reducing and/or elevation suppressing effect according to the present invention can be administered as the pharmaceutical composition having a blood pressure reducing and/or elevation suppressing effect as drugs or quasi-drugs. Preferably, the composition according to the present invention may be administered as a pahrmaceutical composition manufacturable by well known methods. The pharmaceutical composition includes tablet, powdered medicine, granule, fluid, and syrup. The composition according to the present invention as drugs or quasi-drugs may be mixed into food, drink or feed. The composition having a blood pressure reducing and/or elevation suppressing effect, and according to the present invention exhibits the blood pressure reducing and/or elevation suppressing effect, and by oral ingestion, particularly as a food and drink. The intake or dose for obtaining the effect of the present invention is not limited and can be increased or decreased in response to body mass, age, the disease or symptoms of a person or a patient who ingest the composition. Generally, 0.1-40g per day, preferably 0.5-20g per day of the composition may be used for an adult.

For example, the composition having a blood pressure reducing and/or elevation suppressing effect, which is prepared so as to contain a high purified oligosaccharide mass by hydrolyzing coffee extract residues with an acid and/or by heating and which can be added to a liquid coffee, an instant coffee or the like as it is and used but for coffee richer in taste and flavor which coffee inherently possesses can be provided preferably by adding thereto a purified composition that has undergone a.purification treatment such as decolorization, deodorization and deacidification with active carbon, ion-exchange resins, a solvent or the like as the need arises. Herein, liquid coffee includes a commercially available so-called coffee drink or coffee added drink which is put into a can or a so-called PET bottle container. Instant coffee includes so-called soluble powdered coffee obtained by removing water from an extract liquid obtained by extracting roasted and ground coffee with hot water by spraying or freeze-drying, and as a coffee mixed drink, for example, a drink obtained by adding sugar, a creaming powder and the like to soluble powdered coffee and mixed can be mentioned .

### [Example]

The composition having a blood pressure reducing and/or elevation suppressing effect, and the liquid coffee containing the same according to the present invention were prepared in the following manner, and the blood pressure reducing and/or elevation suppressing effect of this drink was examined. The present example specifically explains the embodiments of the present invention and is not intended to limit the scope of the present invention.

### Example 1

### Preparation of Mannooligosaccharide Mass

Roasted and ground coffee obtained by the conventional method was extracted by a commercially employed percolation system and the remaining coffee extraction residues were used.

First, the coffee residues were ground to a particle diameter of about 1 mm to be easily fed into a reactor. Then, a slurry comprising water and ground materials and having a total solid content of about 14 weight% was prepared, and heat-treated in a thermal plug flow reactor having a length of 4 m. The slurry thus obtained was pumped to a plug flow reactor with high-pressure steam at a speed corresponding to a residence time of eight minutes to be maintained at about 210°C with the use of a 6.35 mmϕ orifice. Thereafter, the resulting slurry was allowed to gush forth atmospherically to quickly stop the reaction. The resulting slurry was filtrated to separate a soluble solid content-containing liquid from an insoluble solid content. This soluble solid content-containing liquid was decolorized with active carbon and an adsorptive resin, and furthermore desalted with an ion-exchange resin, then concentrated and dried to obtain a composition comprising a mannose-based oligosaccharide mass comprising 1 to 10 molecules of monosaccharides linked together, said molecules mainly comprising mannose (14% yield).

The DP distribution of oligosaccharide present in the thus obtained composition having a body fat reducing function had DP 1 of 2.4%, DP 2 of 26.6%, DP 3 of 20.2%, DP 4 of 17.8, DP 5 of 10.9%, DP 6 of 8.9, and DP 7 of 6.0%, DP 8 of 3.6%, DP 9 of 1.9% and DP 10 of 1.7%, and the content of a mannose residue in the sugar chain was 90% but the DP distribution and the content of the mannose residue in the sugar chain could be varied depending on the hydrolysis conditions employed. There were mannose and the like as the oligosaccharide having DP 1, mannobiose and the like as the oligosaccharide having DP 2, mannotriose and the like as the oligosaccharide having DP 3, mannotetrose and the like as the oligosaccharide having DP 4, mannopentose and the like as the oligosaccharide of DP 5, mannohexose and the like as the oligosaccharide of DP 6, mannoheptose and the like as the oligosaccharide of DP 7, mannooctose and the like as the oligosaccharide of DP 8, mannononose and the like as the oligosaccharide of DP 9 and mannodecose and the like as the oligosaccharide of DP 10, and the bonding mode was a β-1,4-bonding. With the use of the oligosaccharides thus obtained, the following test was carried out.

### Example 2

### Effect of mannooligosaccharide mass on blood pressure of human by clinical test

The effect of drinking a drink comprising a mannooligosaccharide mass on blood pressure of human was examined.
The test was carried out by double-blind method. The subjects were twenty four males and twenty four females belonging in BMI≧25kg/m² group, and they were grouped into a control group (n=24) and a mannooligosaccharide ingestion group (n=24) so that the BMI of the two groups were about the same. The test drink was liquid coffee and the liquid coffee was prepared by diluting a concentrated coffee extract and artificial sweetener by water and then UHT sterilizing the diluted coffee. The obtained coffee was filled into a 900 mL PET bottle and the filled coffee was used as the control. The test drink for the mannooligosaccharide ingestion group was prepared by adding a liquid comprising mannooligosaccharide into liquid coffee (6 g of mannooligosaccharide per 300 mL). The subjects drank 300 mL test drink per day for twelve weeks, and the blood pressure of the subjects was checked at the start of the test, after four weeks, after eight weeks, and after twelve weeks.

The blood pressure changes based on the start value are illustrated in Figure 1. A significant blood pressure change was not seen in the control group for twelve weeks. On the other hand, the significant blood pressure fall was seen in the mannooligosaccharide ingestion group and this means that ingestion of the drink comprising the mannooligosaccharide mass has a blood pressure reducing effect. Accordingly, the mannooligosaccharide mass has an effect of reducing blood pressure of human.

### Example 3

### Effect of mannooligosaccharide mass on high blood pressure induced rat

### Test animal and breeding condition

Rats developing high blood pressure by salt (salt sensitive high blood pressure rat; Dahl S/Jr Sea) were used for the test. Rats were preliminarily raised for a week for inspection and conditioning and individuals that did not express any defect in the body mass transition and the general condition were used in the test. After the preliminary raising, rats were grouped into a control group consisting of five rats and a mannooligosaccharide ingestion group consisting of five rats so that the average body mass is about the same.

The feed was a mixture of powder feed for common feeding 700 g and cooking oil 300 g, and drinking water comprising 2% of salt was freely ingested from water feeding bottle and thereby high blood pressure was induced. Then purified mannooligosaccharide mass water solution was orally administered three times per day. The dose of mannooligosaccharide was 900 mg per day per individual. On the other hand, the same amount of purified water was orally administered to the control group. The blood pressure was checked at the start of the test, after two weeks, after 3 weeks and after four weeks.

The blood pressure changes based on the start value were shown in Table 1. By administering mannooligosaccharide mass, the significant effect of suppressing blood pressure elevation compared to the control group. Accordingly, administration of the mannooligosaccharide mass has an effect of suppressing blood pressure elevation of rat.

**[Table 1]**

| Table: Change in Systolic Blood Pressure of Rats by administration of mannooligosaccharide Mass | | | |
|---|---|---|---|
| Dosing Period | 2 weeks | 3 weeks | 4 weeks |
| Control (mmHg) | 36.8±5.0 | 22.2±2.8 | 48.4±4.9 |
| Mannooligosaccharide Mass Administered Group (mmHg) | 1.0±8.3^{**} | -5.6±8.7^{*} | 16.6±3.2^{***} |

| | | | |
|---|---|---|---|
| Data are expressed by (average) ± (standard deviation) (n=5) ^{*};p<0.05, ^{**};p<0.01, ^{***};p<0.001/There were significant difference compared to control group. | | | |

### [Industrial Applicability]

The composition having a blood pressure reducing and/or elevation suppressing effect of the present invention comprises a mannose-based oligosaccharide mass which has a blood pressure reducing and/or elevation suppressing effect can be specifically produced by hydrolyzing mannan. By daily ingestion of a food and drink added with the composition having a body fat reducing function according to the present invention, the blood pressure reducing and/or elevation suppressing effect, can be expected. As the raw material for the composition having a blood pressure reducing and/or elevation suppressing effect, for example, coffee beans and coffee extraction residues can be utilized. In other words, a composition having a blood pressure reducing and/or elevation suppressing effect is prepared from coffee extraction residues which have been disposed as waste and can be ingested with food and drink or the like, and accordingly the composition having the blood pressure reducing and/or elevation suppressing effect is a very useful invention from the standpoint of recycling waste resources in addition to an health improving effect.

## Claims

1. A composition having a blood pressure reducing and/or elevation suppressing effect which comprises a mannose-based oligosaccharide mass comprising 1 to 10 molecules of monosaccharides linked together, said molecules mainly comprising mannose.

2. The composition having a blood pressure reducing and/or elevation suppressing effectof claim 1, wherein said mannose-based oligosaccharide mass comprising 1 to 10 molecules of mannose linked together.

3. The composition having a blood pressure reducing and/or elevation suppressing effect of claim 1, wherein said molecules comprising mannose and at least one monosaccharide selected from the group consisting of glucose, galactose and fructose.

4. The composition having a blood pressure reducing and/or elevation suppressing effect of claim 1, wherein the content ratio of said mannose-based oligosaccharide mass is not less than 60 weight% based on the total solid content in the composition.

5. The composition having a blood pressure reducing and/or elevation suppressing effect of any one of claims 1 to 4, wherein the content ratio of a mannose residue in said mannose-based oligosaccharide mass is not less than 70 weight%.

6. The composition having a blood pressure reducing and/or elevation suppressing effect of any one of claims 1 to 5, wherein said mannose-based oligosaccharide mass comprising 2 to 6 molecules of mannose units in the oligosaccharide molecules.

7. The composition having a blood pressure reducing and/or elevation suppressing effect of any one of claims 1 to 6, wherein said mannose-based oligosaccharide mass is obtained by the hydrolysis treatment of mannan.

8. The composition having a blood pressure reducing and/or elevation suppressing effect of claim 7, wherein said mannan is obtained from coffee beans and/or coffee extraction residues.

9. The composition having a blood pressure reducing and/or elevation suppressing effect of any one of claims 1 to 8, wherein said mannose-based oligosaccharide mass is β-1,4-mannooligosaccharide mass.

10. An oral ingestible composition comprising a composition having a blood pressure reducing and/or elevation suppressing effect of any one of claims 1 to 9.

11. The composition of claim 10, wherein said oral ingestible composition is a drink.

12. The composition of claim 11, wherein said drink is a liquid coffee, an instant coffee or a coffee mixed drink. 13 The composition of claim 10, wherein said oral ingestible composition is a food.
